# FASCICULE DE BREVET EUROPEEN

(11) **EP 3 482 700 B1**
(45) Date de publication et mention de la délivrance du brevet: **09.09.2020**
(21) Numéro de dépôt: 18202068.5
(22) Date de dépôt: 23.10.2018
(51) Int. Cl.: A61B 17/16

(54) **PERFORATEUR CRÂNIEN**
SCHÄDELBOHRER
CRANIAL PERFORATOR

(30) Priorité: 13.11.2017 FR 1760655
(43) Date de publication de la demande: 15.05.2019
(73) Titulaire: Vitalys Surgical, 35500 Vitre (FR)
(72) Inventeur: COLLERAIS, Pierre-Yves, 35500 VITRE (FR)
(74) Mandataire: Vidon Brevets & Stratégie

(56) Documents cités:
- WO-A1-2009/012457
- WO-A1-2015/150844
- US-A- 5 330 480

## Description

### 1. Domaine de l'invention

Le domaine de l'invention est celui des instruments chirurgicaux.

Plus précisément, l'invention concerne les outils chirurgicaux qui sont classiquement utilisés par des neurochirurgiens de façon à pratiquer des perforations dans le crâne de patients souffrant de certaines pathologies, après que les tissus recouvrant la zone du crâne devant être perforée aient été préalablement retirés et que celle-ci ait été raclée. Il est généralement pratiqué au minimum trois trépanations, ou perçages du crâne, qui sont suivies d'un sciage entre chaque trou de trépan de façon à retirer un volet crânien et ainsi dégager une voie d'abord.

De tels outils sont habituellement dénommés perforateurs crâniens ou trépans et sont destinés à être raccordés en bout d'un appareillage ou d'un moteur chirurgical, susceptible des les animer d'un mouvement de rotation.

### 2. Art antérieur

Les perforateurs crâniens de l'art antérieur comprennent généralement deux fraises. Une première fraise, ou « petite » fraise est logée à l'intérieur d'une seconde fraise ou « grande » fraise, de telle sorte que la zone de découpe de la première fraise dépasse légèrement de la zone de découpe de la deuxième fraise. Un principe général de leur utilisation pour la perforation d'un crâne est d'appliquer d'abord la première fraise contre la surface du crâne devant être perforée en exerçant une certaine pression, ce qui embraye la première fraise avec le moteur chirurgical auquel le perforateur crânien est relié. Puis, le moteur chirurgical est mis en route, ce qui entraine en rotation la première fraise et la deuxième fraise, la deuxième fraise étant solidaire de la première fraise. Lorsque la première fraise est proche d'avoir traversé la masse osseuse du crâne, la résistance à sa progression diminue, ce qui débraye la première fraise du moteur chirurgical. Bien que le moteur chirurgical continue de tourner, la première fraise et la deuxième fraise s'arrêtent toutes les deux de tourner. Ceci permet notamment de ne pas endommager la dure-mère qui adhère à l'os à l'intérieur de la boîte crânienne.

Les perforateurs crâniens de l'art antérieur sont classiquement constitués d'un nombre important de pièces assemblées entre elles. La multiplicité de ces pièces rend les perforateurs crâniens de l'art antérieur compliqués à fabriquer/usiner et à assembler.

De plus, la multiplicité des pièces implique un cumul de l'incertitude sur le dimensionnement de chaque pièce et un cumul de l'incertitude sur le positionnement de chaque pièce l'une par rapport à l'autre.

Enfin le débrayage de la première fraise avec le moteur chirurgical est assez lent comparé à la vitesse de perçage, ce qui peut aboutir dans certaines conditions à un endommagement plus ou moins important de la dure-mère.

La structure générale d'un de ces perforateurs crâniens a notamment été décrite dans une demande de brevet français publiée sous le numéro FR 2919991. Une telle structure est représentée schématiquement en Figure 1.

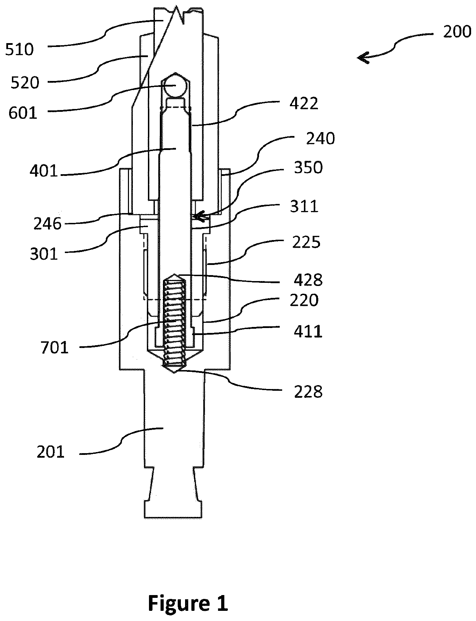

Le perforateur crânien 200 représenté à la Figure 1 comprend un élément tournant 201 formant un carter dont une extrémité peut être raccordée à un moteur chirurgical (non représenté) mis en œuvre afin d'obtenir la mise en rotation de l'élément tournant 201. L'élément tournant 201 présente un premier alésage 220 dont une portion 225 est taraudée.

Une bague intermédiaire 301 est vissée dans la portion taraudée 225 de l'élément tournant 201. La bague intermédiaire 301 est traversée en son milieu par un perçage 311 qui s'étend essentiellement le long de son axe longitudinal. Cette bague intermédiaire 301 est réalisée en bronze car le bronze a une bonne résistance aux frottements et un bon pouvoir lubrifiant.

Une tige de transmission 401 est montée mobile en coulissement à l'intérieur de ce perçage 311 de sorte qu'elle peut y être translatée et pivotée. Une première extrémité de la tige de transmission 401 présente un épaulement 411 dont le diamètre extérieur est supérieur au diamètre du perçage 311 de façon que la tige de transmission 401 ne puisse être translatée à l'intérieur du perçage 311 au-delà d'une certaine mesure.

Une première fraise 510 est vissée à une portion filetée 422 de la deuxième extrémité de la tige de transmission 401.

Une bille de compression 601 est interposée entre la première fraise 510 et la deuxième extrémité de la tige de transmission 401.

Une deuxième fraise 520, à l'intérieur de laquelle est logée la première fraise 510, est liée en rotation à la première fraise 510. À cet effet, les première 510 et deuxième 520 fraises présentent respectivement des éléments de forme complémentaire (non représentés) susceptibles d'entrer en prise de façon que la rotation de la première fraise 510 entraîne la rotation de la deuxième fraise 520.

Des moyens d'embrayage 350 entre la première fraise 510 et la bague intermédiaire 301 permettant dans une position embrayée que la première fraise 510 soit liée en rotation avec la bague intermédiaire 301 quand l'élément tournant 201 est mis en rotation et, dans une position de repos que la première fraise 510 ne soit pas liée en rotation avec la bague intermédiaire 301 quand l'élément tournant 510 est mis en rotation ;

Un ressort de compression 701 est disposé entre un logement 428 ménagé à cet effet à la première extrémité de la tige de transmission 401 et un réceptacle 228 ménagé à cet effet en bout du premier alésage 220 de l'élément tournant 201. Il permet de passer de la position embrayée vers la position de repos quand la force axiale exercée sur la première fraise n'est pas suffisante.

L'élément tournant 201 présente un deuxième alésage 240 à l'intérieur duquel la deuxième fraise 520 vient se loger. Le deuxième alésage 240 présente un épaulement 246 le long duquel la deuxième fraise 520 est susceptible de venir en butée.

Un perforateur crânien tel que celui représenté à la Figure 1 présente cependant de nombreux désavantages.

Il comprend en tout sept pièces différentes : une première fraise 510, une deuxième fraise 520, une bille de compression 601, une tige de transmission 401, une bague intermédiaire 301, un ressort de compression 701 et un élément tournant 201. Il en résulte une complexité de fabrication/usinage et une complexité d'assemblage des pièces entre elles. Il en résulte également un cumul possible des erreurs de dimensionnement sur chacune des pièces, provoquant une déviation de la chaîne des cotes des différentes pièces du perforateur crânien entre elles. Par ailleurs, en dehors des erreurs de dimensionnement, la déviation de la chaîne des cotes peut également être accentuée par un mauvais assemblage. Un mauvais assemblage peut notamment résulter d'un vissage incomplet entre des pièces. Or, le perforateur crânien de la Figure 1 comprend deux vissages de pièces entre elles. Les conséquences d'une déviation trop importante de la chaîne des cotes des pièces du perforateur crânien peuvent être un manque de fiabilité du perforateur crânien pour le perçage du crâne, voire si la déviation est trop importante, une dangerosité du perforateur crânien pour le perçage du crâne.

La bague intermédiaire 301 en bronze est, en particulier, peu avantageuse. Comme déjà évoqué, elle est source d'une possible erreur de positionnement dans le perforateur crânien lors de l'assemblage, notamment à cause d'un possible vissage incomplet à l'intérieur de l'élément tournant 201. De plus, elle présente une surface importante de frottement avec la tige de transmission 401. En outre, le bronze n'est pas une matière biocompatible et il est préférable de ne pas l'utiliser, des microparticules pouvant être projetées lorsque le perforateur crânien perfore un crâne. Enfin, comme le perforateur crânien de la figure 1 peut facilement être démonté par dévissage et/ou désengagement des différentes pièces, il peut en fin de compte être réutilisé plusieurs fois après stérilisation en autoclave de l'ensemble des pièces le constituant. Or bien que stérilisées en autoclave, les fraises du perforateur crânien peuvent toujours transmettre le prion de Creutzfeldt-Jakob. Il est donc nécessaire de garantir l'usage unique du perforateur crânien.

On connaît aussi selon WO 2015/150844 A1 un perforateur crânien comprenant un élément tournant actionné par un élément d'entraînement, une première fraise et une deuxième fraise pourvues chacune de dents à leur extrémité tournant autour d'un même axe et pouvant se déplacer en translation le long de celui-ci, la première fraise étant logée à l'intérieur de la deuxième fraise, et des moyens d'embrayage permettant, dans une position embrayée, que la première fraise soit liée en rotation avec l'élément tournant et, dans une position de repos, que la première fraise ne soit pas liée en rotation avec celui-ci. Un tel perforateur comprend aussi des moyens de désengagement de la position embrayée vers la position de repos, un carter enveloppant en partie la deuxième fraise, la deuxième fraise étant liée au carter par une liaison pivot glissant et comprenant une surface d'appui apte à venir en butée contre le carter lorsque la deuxième fraise est animée d'un mouvement de translation s'éloignant de l'élément tournant. Un tel perforateur crânien présente toutefois également au moins certains des inconvénients indiqués ci-dessus.

### 3. Objectifs de l'invention

L'invention a pour objectifs de pallier au moins certains des inconvénients de l'art antérieur.

Un objectif de l'invention est de fournir un perforateur crânien qui ait une structure optimisée et qui soit fiable et sûr lors du perçage d'un crâne.

En particulier, un objectif de l'invention est de fournir un perforateur crânien qui contienne un nombre restreint de pièces.

Un autre objectif de l'invention est, selon au moins certains modes de réalisation avantageux, de fournir un perforateur crânien comprenant des pièces simples à fabriquer/usiner.

Un autre objectif est, selon au moins certains modes de réalisation avantageux, de fournir un perforateur crânien comprenant des pièces faciles à assembler les unes aux autres.

Un autre objectif est, selon au moins certains modes de réalisation avantageux, de fournir un perforateur crânien dont la structure garantit un usage unique.

Un autre objectif de l'invention est, selon au moins certains modes de réalisation avantageux, de fournir un perforateur crânien ayant un débrayage amélioré.

### 4. Exposé de l'invention

L'invention concerne un perforateur crânien tel que défini dans la revendication 1, comprenant : un élément tournant,
une première fraise, une deuxième fraise, des moyens d'embrayage entre la première fraise et l'élément tournant, des moyens de désengagement entre la première fraise et l'élément tournant et un carter.

L'élément tournant est apte à être fixé à un élément d'entraînement.

La première fraise et la deuxième fraise sont aptes à tourner autour d'un même axe et à se mouvoir en translation le long de cet axe. Elles présentent chacune en l'une de leurs extrémités une pluralité de dents. La première fraise est logée à l'intérieur de la deuxième fraise. La première fraise comprend au moins une première surface d'appui apte à venir en butée contre la deuxième fraise lorsque la première fraise est mise en rotation autour de son axe et, au moins une deuxième surface d'appui apte à venir en butée contre la deuxième fraise lorsque la première fraise est animée d'un mouvement de translation s'éloignant de l'élément tournant selon son axe.

Les moyens d'embrayage disposés sur la première fraise et l'élément tournant permettent dans une position embrayée que la première fraise soit liée en rotation avec l'élément tournant quand l'élément tournant est mis en rotation et, dans une position de repos que la première fraise ne soit pas liée en rotation avec l'élément tournant quand l'élément tournant est mis en rotation.

Les moyens de désengagement permettent un désengagement de la position embrayée vers la position de repos.

Le carter est fixé en l'une de ses extrémités à l'élément tournant et enveloppe en partie la deuxième fraise. La deuxième fraise est liée au carter par une liaison pivot glissant et comprend une surface d'appui apte à venir en butée contre le carter lorsque la deuxième fraise est animée d'un mouvement de translation s'éloignant de l'élément tournant selon son axe.

Ainsi, le perforateur crânien selon l'invention ne comprend pas de bague intermédiaire, ni de tige de transmission.

Pour assurer des fonctions analogues à celles assurées par ces pièces dans le perforateur crânien selon l'art antérieur, le perforateur crânien selon l'invention comprend notamment une liaison pivot glissant liant le carter à la deuxième fraise. Cette liaison pivot glissant permet d'assurer une rotation parfaite de la seconde fraise autour de son axe ainsi que de guider la seconde fraise en translation selon son axe. La première surface d'appui de la première fraise apte à venir en butée contre la deuxième fraise lorsque la première fraise est mise en rotation permet de lier en rotation la première et la deuxième fraise. La deuxième surface d'appui de la première fraise apte à venir en butée contre la deuxième fraise lorsque la première fraise est animée d'un mouvement de translation s'éloignant de l'élément tournant selon son axe et la surface d'appui de la seconde fraise apte à venir en butée contre le carter lorsque la deuxième fraise est animée d'un mouvement de translation s'éloignant de l'élément tournant selon son axe permettent de limiter l'amplitude de la translation des première et deuxième fraises selon leur axe à une certaine mesure.

Les moyens d'embrayage sont de plus directement disposés entre l'élément tournant et la première fraise. Aucune pièce intermédiaire n'est donc disposée entre l'élément tournant et la première fraise pour assurer leur embrayage.

Le carter du perforateur crânien selon l'invention permet d'assurer que la rotation de la deuxième fraise et de la première fraise ont lieu précisément autour de leur axe de rotation. Le carter permet également de limiter l'amplitude d'un mouvement de translation des première et deuxième fraises selon leur axe à une certaine mesure.

Préférentiellement, le carter comprend une section cylindrique d'un diamètre supérieur au diamètre d'une section cylindrique de la deuxième fraise. La liaison pivot glissant est avantageusement formée par au moins une lèvre faisant saillie sur une surface extérieure de la section cylindrique de la deuxième fraise ou sur une surface intérieure de la section cylindrique du carter. Une telle liaison pivot glissant présente comme intérêt de présenter une faible surface de frottement.

La liaison pivot glissant peut notamment être formée par deux lèvres faisant saillie sur une surface extérieure de la section cylindrique de la deuxième fraise ou sur une surface intérieure de la section cylindrique du carter.

Préférentiellement, la surface d'appui de la deuxième fraise apte à venir en butée contre le carter lorsque la deuxième fraise est animée d'un mouvement de translation s'éloignant de l'élément tournant selon son axe est formée par un épaulement de la deuxième fraise, l'épaulement de la deuxième fraise étant apte à venir en butée contre un épaulement du carter.

Avantageusement, le carter est fixé à l'élément tournant par un emboitage élastique irréversible du carter avec l'élément tournant. On entend par « emboitage élastique » un mode d'assemblage dans lequel les éléments sont déformés lors de l'introduction de l'un dans l'autre. On entend par « irréversible » le fait que lorsque le carter et l'élément tournant sont emboités élastiquement l'un dans l'autre, un désassemblage ne peut être obtenu que par rupture du carter et/ou de l'élément tournant. L'emboitage élastique irréversible du carter avec l'élément tournant permet de rendre indémontable le perforateur crânien. Ainsi, le perforateur crânien ne peut pas être nettoyé, décontaminé et/ou stérilisé pour une seconde réutilisation. L'emboitage élastique irréversible du carter avec l'élément tournant garantit donc un usage unique du perforateur crânien selon l'invention. L'emboîtage élastique irréversible peut notamment être obtenu grâce à un système de rainure-languette, dont les éléments sont respectivement disposés sur l'élément tournant et sur le carter, la languette venant s'emboîter dans la rainure.

L'élément tournant est apte à être fixé à un élément d'entraînement, tel un moteur chirurgical. L'élément tournant possède notamment à l'une de ses extrémités un embout adapté pour être fixé à un élément d'entraînement. L'élément d'entraînement présente à l'autre de ses extrémités des moyens d'embrayage permettant un embrayage avec la première fraise.

La première fraise et la deuxième fraise présentent chacune à l'une de leurs extrémités une pluralité de dents. La première fraise est logée à l'intérieur de la deuxième fraise.

Selon une caractéristique avantageuse, chacune des dents présente une face de coupe qui s'étend le long d'un plan essentiellement parallèle et décalé de l'axe de la première fraise (respectivement deuxième fraise). Cette géométrie particulière permet de faciliter la formation de copeaux fins au cours de la perforation du crâne, et d'en améliorer l'écoulement et l'évacuation. Ces copeaux pourront par exemple être réutilisés afin de combler les espaces vides laissés entre le volet crânien et le crâne au moment de la fermeture de celui-ci et permettre de recoloniser l'os du crâne et d'obtenir une fusion à moyen terme du volet et du crâne.

Selon une autre caractéristique avantageuse de l'invention, chacune des dents présente des zones tranchantes, les zones tranchantes formant saillies et s'étendant essentiellement depuis la périphérie de l'extrémité présentant une pluralité de dents de la première fraise (respectivement deuxième fraise) en direction d'une zone centrale évidée. Cette géométrie particulière permet d'attaquer la surface d'un crâne à percer par la périphérie de la première fraise (respectivement deuxième fraise) de façon à ce qu'en fin de perçage, une portion d'os non taillé de forme circulaire, encore appelée pastille d'os, correspondant à la zone centrale évidée, persiste. La persistance d'une telle pastille d'os en fin de perçage permet de protéger la dure-mère recouvrant la surface intérieure du crâne et ainsi de prévenir son endommagement.

Préférentiellement, la première fraise présente une pointe de centrage. La mise en œuvre d'une telle pointe de centrage permet de faciliter l'amorçage du perçage et en particulier d'éviter le ripage du trépan. Ceci permet tant d'améliorer la précision du perçage, la sécurité et le confort d'utilisation.

La première fraise comprend une première surface d'appui apte à venir en butée contre la deuxième fraise lorsque la première fraise est mise en rotation autour de l'axe. Ceci permet la mise en rotation de la deuxième fraise quand la première fraise est mise en rotation. Une telle butée peut être formée par un système ergot-entaille, dont les éléments sont disposés respectivement sur la première fraise et sur la deuxième fraise, l'ergot traversant l'entaille et pouvant venir en butée contre elle.

La première fraise comprend une deuxième surface d'appui apte à venir en butée contre la deuxième fraise lorsque la première fraise est animée d'un mouvement de translation s'éloignant de l'élément tournant selon son axe. Ceci permet de limiter l'amplitude d'un mouvement de translation de la première fraise par rapport à la deuxième fraise. Une telle butée peut être formée par un épaulement de la première fraise apte à venir en butée contre un épaulement de la deuxième fraise.

Les moyens d'embrayage sont disposés sur la première fraise et l'élément tournant. Ils permettent dans une position embrayée que la première fraise soit liée en rotation avec l'élément tournant quand l'élément tournant est mis en rotation et, dans une position de repos que la première fraise ne soit pas liée en rotation avec l'élément tournant quand l'élément tournant est mis en rotation.

Préférentiellement les moyens d'embrayage entre la première fraise et l'élément tournant comprennent au moins un plan incliné et une surface de blocage disposés sur l'un de ladite première fraise ou dudit élément tournant et, au moins un doigt présentant une surface d'appui susceptible de venir en contact avec la surface de blocage dans la position d'embrayage, ledit au moins un doigt étant disposé sur l'autre de ladite première fraise ou dudit élément tournant. Ces moyens d'embrayage permettent un embrayage progressif de la première fraise avec l'élément tournant. Autrement dit, le passage de la première fraise en position embrayée peut se faire de manière douce et sans à coup.

Les moyens de désengagement permettent de maintenir la première fraise dans une position de repos quand la force axiale exercée sur la première fraise n'est pas suffisante.

Selon une caractéristique préférentielle de l'invention les moyens de désengagement comprennent un moyen de rappel, tel un ressort de compression, qui tend à ramener la première fraise dans la position de repos. Le moyen de rappel est disposé entre la première fraise et l'élément tournant.

Avantageusement, les moyens de désengagement comprennent en outre ladite au moins première surface d'appui de ladite première fraise et, au moins une rampe disposée sur ladite deuxième fraise et inclinée par rapport à son axe, ladite au moins première surface d'appui de ladite première fraise apte à venir en butée contre ladite deuxième fraise, étant apte à former une liaison glissière lorsque ladite première fraise est mise en rotation. La transmission du couple entre la première fraise et la deuxième fraise au niveau d'une liaison glissière inclinée par rapport à l'axe des fraises permet ainsi de créer une composante axiale de force facilitant le débrayage de la première fraise, c'est à dire son retour rapide en position de repos.

Avantageusement, l'angle formé entre ladite au moins une rampe et ledit axe est compris entre 15° et 75° par rapport audit axe, préférentiellement entre 30° et 60° par rapport audit axe, de manière davantage préférée égale à 45° par rapport audit axe.

Selon un mode de réalisation particulier, la première fraise comprend deux premières surfaces d'appui aptes à former deux liaisons glissières avec deux rampes disposées sur la deuxième fraise. Les deux rampes, respectivement les deux surfaces d'appui ne se superposent pas par rotation d'un angle de 180° selon ledit axe. Ceci permet de garantir un positionnement unique des dents de la première fraise par rapport aux dents de la deuxième fraise.

Selon une caractéristique préférentielle de l'invention, le perforateur crânien ne comprend pas de zone filetée. Ceci permet d'éviter toute erreur, notamment un mauvais vissage, lors de l'assemblage des pièces du perforateur crânien.

L'invention concerne également un kit comprenant l'ensemble des pièces du perforateur crânien selon l'invention.

L'invention concerne aussi un procédé de montage d'un kit comprenant l'ensemble des pièces du perforateur crânien selon l'invention.

### 5. Liste des figures

La Figure 1, représente un perforateur crânien de l'art antérieur, tel que celui décrit dans la demande de brevet français déposé par la Déposante et publié sous le numéro FR 2919991.

L'invention, ainsi que les différents avantages qu'elle présente, seront plus facilement compris grâce à la description qui va suivre d'un mode non limitatif de réalisation de celle-ci, donnée en référence aux dessins présentés aux Figures 2-9, dans lesquels :
La Figure 2 représente une vue en perspective et éclaté d'un perforateur crânien selon l'invention.
La Figure 3 représente une vue de face du perforateur crânien de la Figure 2. La Figure 3bis est une coupe de la Figure 3 passant par l'axe de rotation du dispositif.
La Figure 4 et la Figure 5 représentent l'élément tournant du perforateur crânien selon la Figure 2. La Figure 4 est une vue de face. La Figure 5 est une vue de dessus.
Les Figures 6 et 7 représentent la première fraise du perforateur crânien selon la Figure 2. La Figure 6 est une vue de face. La Figure 7 est une vue de côté.
La Figure 8 représente la deuxième fraise, vue de face, du perforateur crânien selon la Figure 2.
La Figure 9 représente le carter du perforateur crânien selon la Figure 2.

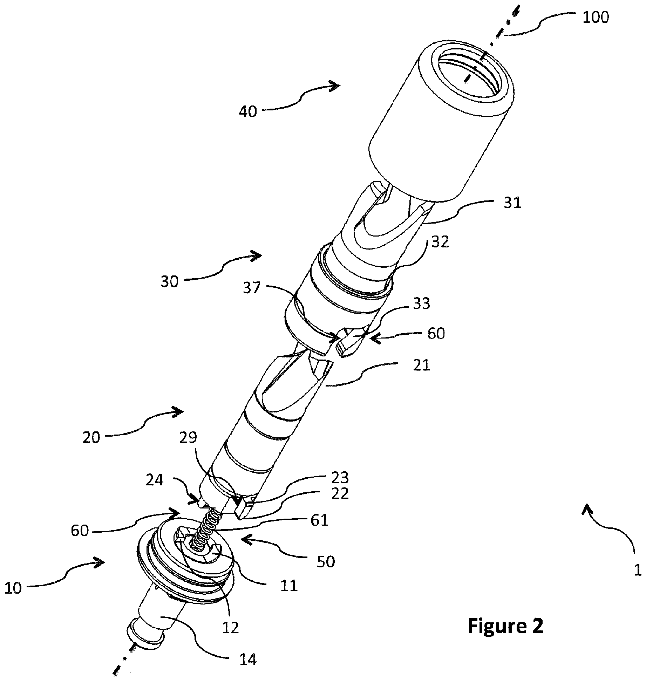

Dans les Figures 2-9, le perforateur crânien et/ou les pièces le constituant sont orientés de telle sorte que l'élément tournant soit en bas et les dents de la première fraise et de la deuxième fraise soient en haut. Dans la description ci-dessous, « en haut », « partie supérieure », « en bas » et « partie inférieure » font référence à cette convention. L'axe des fraises selon cette convention est donc un axe vertical dans le plan des dessins.

### 6. Description détaillée d'un mode de réalisation

### Structure du perforateur crânien

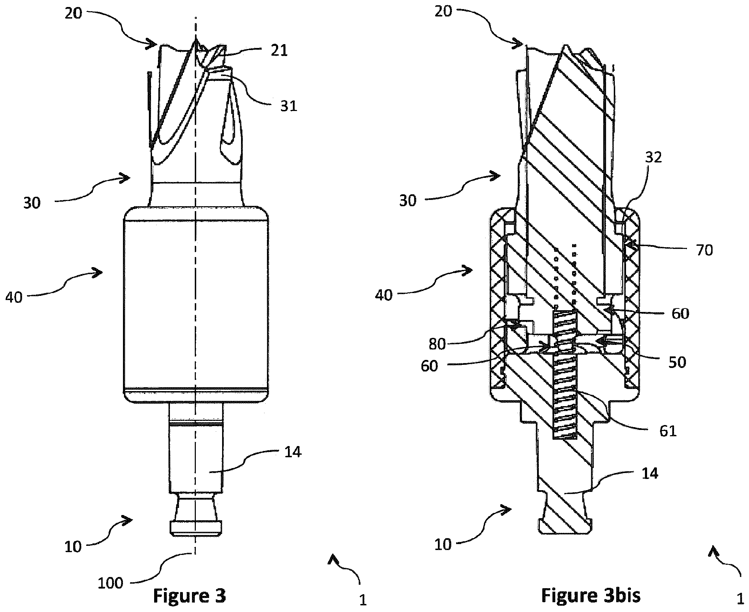

En référence aux figures 2, 3 et 3bis, le perforateur crânien 1 comprend cinq pièces : un élément tournant 10, un ressort de compression 61, une première fraise 20, une deuxième fraise 30 et un carter 40.

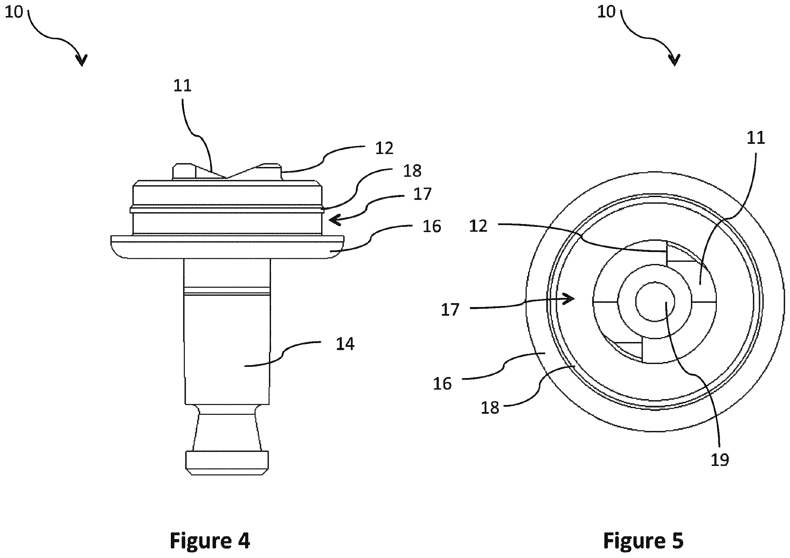

En référence en particulier aux Figures 4 et 5, l'élément tournant 10 comprend en son extrémité inférieure une connexion 14, de type HUDSON, permettant de le raccorder à un moteur chirurgical (non représenté) mis en œuvre afin d'obtenir sa mise en rotation. On note qu'un tel moteur chirurgical (non représenté) peut mettre en rotation l'élément tournant 10 au moyen d'énergie électrique provenant du secteur ou stockée dans des batteries, ou d'énergie pneumatique.

L'élément tournant 10 comprend une plate-forme 16, circulaire, surmontée d'un plateau 17 de forme essentiellement cylindrique. La plate-forme 16 a un diamètre plus important que le plateau 17. Elle permet de faire butée avec le carter 40 quand celui-ci est monté. Le plateau 17 comprend une languette circulaire 18 faisant saillie à la surface du cylindre.

Le plateau 17 est surmonté en sa partie centrale par deux plans inclinés 11 intercalés entre deux cercles concentriques de diamètre inférieur à celui du plateau 17. Chaque plan incliné 11 débouche sur la surface supérieure du plateau 17 et se prolonge par une surface de blocage 12, qui s'étend essentiellement à la verticale, correspondant au début de l'autre plan incliné 11.

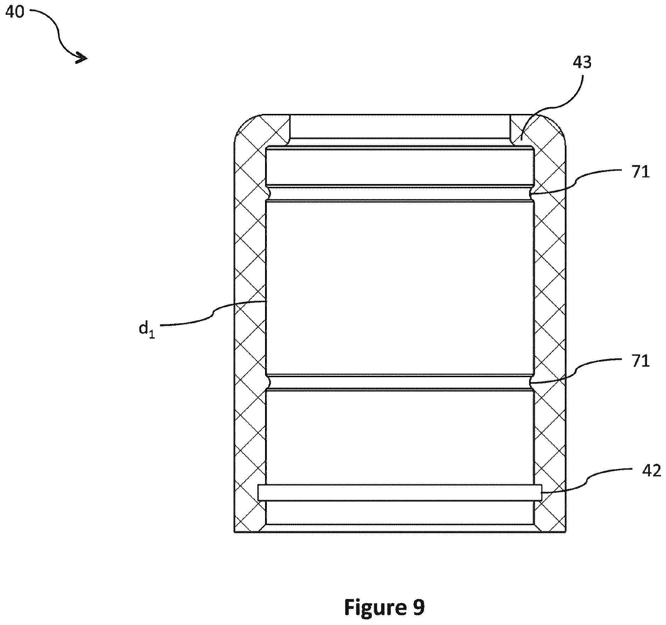

En référence à la Figure 9, le carter 40 a essentiellement la forme d'un cylindre creux ayant une section cylindrique de diamètre interne d₁ sensiblement égal à celui du plateau 17. Il comprend en sa partie basse sur sa surface interne une rainure circulaire 42 de forme complémentaire à la languette circulaire 18 de l'élément tournant 10. Lors de montage du perforateur crânien 1, le carter 40 est fixé à l'élément tournant 10 à l'aide d'une presse. La languette circulaire 18 vient alors s'emboîter dans la rainure circulaire 42. Ceci permet un emboitage élastique irréversible entre le carter 40 et l'élément tournant 10. Grâce à cet emboîtage élastique irréversible, le perforateur crânien 1 ne peut pas être démonté et ne peut donc pas être nettoyé, décontaminé et/ou stérilisé dans la perspective d'une éventuelle seconde réutilisation. L'emboitage élastique irréversible du carter 40 avec l'élément tournant 10 garantit donc un usage unique du perforateur crânien 1.

Le carter 40 comprend en sa partie médiane deux lèvres 71 circulaires faisant saillie sur sa surface interne. Les deux lèvres 71 circulaires permettent d'assurer une liaison de type pivot glissant 70 entre le carter 40 et la deuxième fraise 30. La liaison pivot glissant 70 permet d'assurer une rotation parfaite de la deuxième fraise 30 autour de son axe 100 ainsi que de guider la deuxième fraise 30 en translation selon son axe 100.

Le carter 40 comprend également en sa partie haute un épaulement 43 de plus petit diamètre que le diamètre du reste de la section cylindrique du carter 40. L'épaulement 43 permet de former butée avec la deuxième fraise 30 afin de limiter l'amplitude d'un mouvement de translation de la deuxième fraise 30 selon son axe 100 à une certaine mesure.

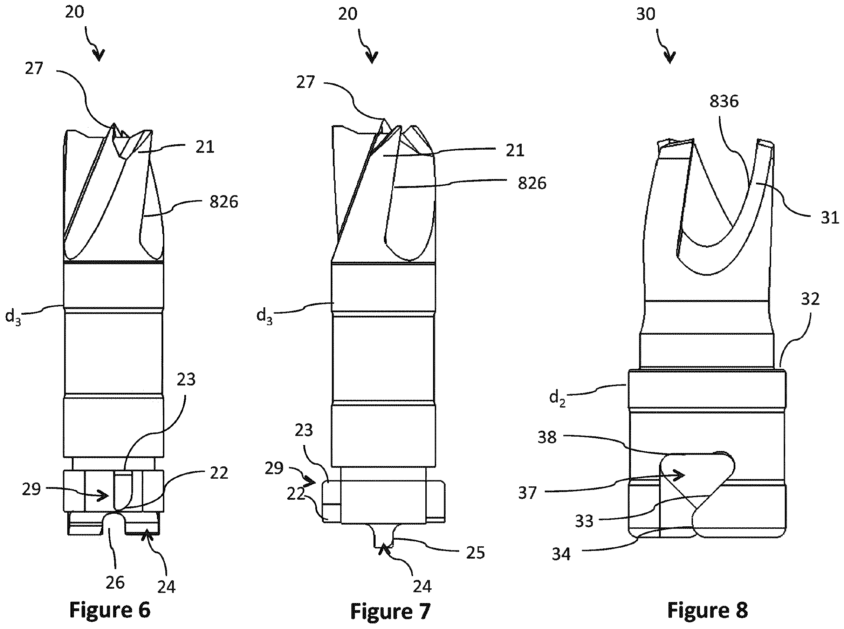

En référence aux Figures 6-8, les première 20 et deuxième 30 fraises présentent chacune en partie supérieure trois dents 21, 31 réparties à 120°. Les dents 21, 31 présentent des zones tranchantes 826, 836 formant saillie et s'étendant essentiellement depuis la périphérie en direction d'une zone centrale évidée. La première fraise 20 est logée à l'intérieur de la deuxième fraise 30 de telle façon que sa zone de coupe dépasse de la zone de coupe de la deuxième fraise 30. La partie supérieure de la première fraise présente en outre une pointe de centrage 27, de forme pyramidale. Le carter 40 ne recouvre évidemment pas les zones de coupe de la première fraise 20 et de la deuxième fraise 30.

Différents lots de premières fraises 20 et de deuxièmes fraises 30 peuvent avoir des zones de coupe différentes de façon à permettre un usage sur des groupes de population différents, notamment des adultes, des adolescents ou des enfants.

La partie médiane et la partie inférieure de la deuxième fraise 30 sont enveloppées par le carter 40. Elles ont essentiellement la forme d'un cylindre creux ayant une section cylindrique de diamètre externe d₂, avec d₂ < d₁. Les lèvres 71 sont en contact avec les parois du cylindre de diamètre externe d₂ et forment ainsi la liaison pivot glissant 70. Les zones de frottement de la liaison pivot glissant 70 sont donc ici limitées comparativement à la technique de l'art antérieur décrite ci-dessus. A noter que sur la Figure 8, un rétrécissement en section médiane est représenté. Ce rétrécissement permet de réduire la quantité de matériau utilisé pour fabriquer la deuxième fraise 30 sur une partie non-fonctionnelle de celle-ci.

L'épaulement 32 marque la frontière entre la partie médiane et la partie supérieure de la deuxième fraise 30. La partie médiane a un diamètre plus grand que le bas de la partie supérieure. L'épaulement 32 de la deuxième fraise permet de former butée avec l'épaulement 43 du carter 40 et de limiter ainsi l'amplitude d'un mouvement de translation de la deuxième fraise 30 selon son axe 100 à une certaine mesure.

Deux encoches 37, de forme essentiellement triangulaire, sont percées depuis la partie inférieure de la deuxième fraise 30 vers la partie médiane de la deuxième fraise 30. Chaque encoche 37 comprend une rampe 33 inclinée par rapport à l'axe 100 et terminant en bas de la rampe sur un arrondi 34, ainsi qu'une surface plane 38 perpendiculaire à l'axe 100.

La rampe 33 permet de former une liaison glissière 80 avec la première fraise 20. La rampe 33 permet ainsi de créer une composante axiale de force facilitant le débrayage de la première fraise 20 lors de la transmission du couple de rotation entre la première fraise 20 et la deuxième fraise 30. La rampe 33 peut être inclinée d'un angle compris entre 15° et 75° par rapport à l'axe 100. Préférentiellement, la rampe 33 peut être inclinée d'un angle compris entre 30° et 60° par rapport à l'axe 100. En particulier, un angle égal à 45° par rapport à l'axe 100 est particulièrement avantageux puisqu'il permet à la fois une bonne transmission du couple de rotation tout en facilitant le débrayage de la deuxième fraise.

La surface plane 38 perpendiculaire à l'axe 100 permet de faire butée avec la première fraise 20 afin de limiter l'amplitude d'un mouvement de translation de la première fraise 20 selon son axe 100 à une certaine mesure.

L'extrémité inférieure de la deuxième fraise 30 repose en butée sur le plateau 17 de l'élément tournant 10. L'extrémité inférieure peut être chanfreinée afin de limiter les frottements avec le plateau 17, lorsque que l'élément tournant 10 est mis en rotation mais pas la deuxième fraise 30.

La partie médiane de la première fraise 20 a essentiellement la forme d'un cylindre creux ayant une section cylindrique de diamètre externe d₃, inférieur au diamètre interne des sections médiane et inférieur de la deuxième fraise 30. A noter que sur la Figure 6 et la Figure 7, un rétrécissement en section médiane est représenté. Ce rétrécissement permet de réduire la quantité de matériau utilisé pour fabriquer la première fraise 20 sur une partie non-fonctionnelle de celle-ci.

La partie inférieure de la première fraise 20 comprend deux ergots 29 faisant saillie radialement de la première fraise 20 et traversant les deux encoches 37 de la deuxième fraise 30. Chaque ergot 29 comprend une première surface d'appui 22 apte à venir en butée contre la rampe 33 de la deuxième fraise 30, lorsque la première fraise 20 est mise en rotation. La première surface d'appui 22 est arrondie et est apte à venir en contact avec la rampe 33 formant ainsi une liaison glissière. Chaque ergot 29 comprend également une deuxième surface d'appui 23 apte à venir en butée contre la deuxième fraise 30 lorsque la première fraise est animée d'un mouvement de translation s'éloignant de l'élément tournant 10 selon l'axe 100. La deuxième surface d'appui 23 est sensiblement perpendiculaire à l'axe 100 et est apte à former butée avec la surface plane 38 de l'encoche 37.

Les deux ergots 29, respectivement les deux encoches 37, sont disposés de manière antisymétriques sur ladite première fraise 20, respectivement sur ladite deuxième fraise 30, et sont aptes à former ainsi deux à deux, deux liaisons glissières. Ceci permet de loger la première fraise 20 d'une unique manière par rapport à la deuxième fraise 30 afin de garantir notamment un bon positionnement relatif des dents 21 de la première fraise 20 par rapport aux dents 31 de la deuxième fraise 30.

La partie inférieure de la première fraise 20 comprend également deux doigts 24 s'étendant axialement vers le bas. Les deux doigts 24 sont aptes à glisser le long des plans inclinés 11 de l'élément tournant et à être bloqués par les surfaces de blocage 12. Plus précisément, chaque doigt 24 comprend une surface d'appui 25 essentiellement verticale et arrondie en son extrémité basse permettant le glissement du doigt 24 le long du plan incliné 11 puis la formation d'une butée contre la surface de blocage 12.

Les deux doigts 24 de la première fraise 20 et les deux plans inclinés 11 comprenant une surface de blocage 12 de l'élément tournant 10 forment deux à deux les moyens d'embrayage 50 de la première fraise 20 avec l'élément tournant 10. Les moyens d'embrayage 50 permettent dans une position embrayée que la première fraise 20 soit liée en rotation avec l'élément tournant 10 quand l'élément tournant 10 est mis en rotation et, dans une position de repos que la première fraise 20 ne soit pas liée en rotation avec l'élément tournant 10 quand l'élément tournant 10 est mis en rotation. Les moyens d'embrayage 50 sont progressifs et permettent ainsi de prévenir de tout à coup, lors de l'embrayage.

Les deux doigts 24, respectivement les deux plans inclinés 11 comprenant une surface de blocage 12, sont disposés de manière symétrique par rapport à l'axe 100 sur la première fraise 20, respectivement l'élément tournant 10.

Une cavité 26 est aménagée le long de l'axe 100 entre les doigts 24 de la première fraise et, une cavité 19 est aménagée entre les plans inclinés 11 le long de l'axe 100, permettant d'y disposer le ressort de compression 61. Le ressort de compression 61 et les liaisons glissières 80 entre la première fraise 20 et la deuxième fraise 30 lorsque la première fraise est mise en rotation constituent les moyens de désengagement 60 du perforateur crânien 1. Ils permettent le débrayage rapide de la première fraise 20 quand la force axiale exercée sur elle n'est plus suffisante, c'est à dire un retour de la première fraise à une position de repos.

### Montage du perforateur crânien

La structure même des pièces du perforateur crânien 1 permet un assemblage simple ne nécessitant pas d'outils spécifique autre qu'une presse pour être monté. La structure même des pièces du perforateur crânien 1 garantit également qu'aucune erreur ou imprécision ne peut résulter de l'assemblage étant donné que les pièces s'enfilent simplement les unes avec les autres et qu'aucune pièce ne présente de filetage.

Pour assembler le perforateur crânien, il faut rapprocher l'élément tournant 10 et la première fraise 20 puis insérer le ressort de compression 61 dans les cavités 19 et 26 ménagées à cet effet. Puis, il faut enfiler la deuxième fraise 30 par dessus la première fraise 20 en veillant à ce que les ergots 29 de la première fraise 20 soient bien engagés dans les encoches 37 de la deuxième fraise 30. Ensuite, le carter 40 peut être enfilé par dessus la deuxième fraise 30, un emboitage élastique irréversible du carter 40 dans l'élément tournant 10 pouvant être obtenu par pressage.

### Fonctionnement du perforateur crânien

De façon à pratiquer une perforation dans un crâne au moyen d'un perforateur crânien selon l'invention, un chirurgien applique la première fraise 20 contre la surface du crâne devant être perforée en exerçant une certaine pression.

Le chirurgien met ensuite en route le moteur chirurgical auquel le perforateur est relié par l'intermédiaire d'un mandrin de type HUDSON de façon à ce que l'élément tournant 10 soit entraîné à rotation.

Les doigts 24 de la première fraise glissent alors le long des plans inclinés 11 de l'élément tournant 10 depuis leur partie la plus élevée jusqu'à ce que la surface d'appui 25 des doigts 24 forme butée avec les surfaces de blocage 12 des plans inclinés 11. Ceci permet un embrayage progressif de la première fraise 20 avec l'élément tournant 10. Le couple de l'élément tournant 10 est alors transmis à la première fraise 20.

Une fois la première fraise 20 mise en rotation, chaque première surface d'appui 22 des ergots 29 vient en contact avec la rampe 33 de chaque encoche 37 de la deuxième fraise 30. La transmission du couple entre la première fraise 20 et l'élément tournant 10 s'effectue tant qu'il subsiste une épaisseur d'os devant la pointe de centrage 27 de la première fraise 20.

Lorsqu'il n'y a plus d'épaisseur d'os devant la pointe de centrage 27 de la première fraise 20, il n'y a donc plus de force axiale qui pousse la première fraise 20 contre l'élément tournant 10 . La force de réaction du ressort de compression 61 va alors permettre de pousser la première fraise 20 la faisant remonter de 1mm environ (première course de débrayage). Pour aider le ressort de compression à faire remonter davantage la première fraise 20, les liaisons glissières 80, formées par le contact des premières surfaces d'appui 22 des ergots 29 avec les rampes 33 des encoches 37, vont favoriser durant cette première course de débrayage l'éjection de la première fraise 20. En effet, les rampes 33 étant inclinées par rapport à l'axe 100, elles permettent d'une part la transmission du couple de la première fraise 20 à la deuxième fraise 30 et d'autre part de créer une composante de force axiale s'ajoutant à la réaction du ressort de compression 61 tendant à faire remonter la première fraise 20 par rapport à l'élément tournant 10, pour un débrayage progressif et plus rapide de la première fraise 20.

Dès que la première fraise 20 n'est plus embrayée avec l'élément tournant 10, la première fraise 20 s'immobilise et ne transmet donc plus de couple à la deuxième fraise 30. La perforation s'arrête, même si le moteur chirurgical continue de tourner. Le praticien peut retirer le perforateur crânien 1 du crâne.

Le praticien n'aura plus qu'à retirer au moyen d'une spatule la capsule osseuse formée. On note que la deuxième fraise 30 permet, au moment où la première fraise 20 traverse la masse osseuse du crâne, d'éviter qu'elle continue sa progression vers l'intérieur du crâne et endommage la dure-mère.

## Revendications

1. Perforateur crânien (1) comprenant :
- un élément tournant (10) apte à être fixé à un élément d'entraînement ;
- une première fraise (20) et une deuxième fraise (30), aptes à tourner autour d'un même axe (100) et à se mouvoir en translation le long dudit axe (100) l'une par rapport à l'autre ainsi que chacune par rapport audit élément tournant (10), et présentant chacune en l'une de leurs extrémités une pluralité de dents (21; 31),
ladite première fraise (20) étant logée à l'intérieur de ladite deuxième fraise (30),
- des moyens d'embrayage (50) disposés sur ladite première fraise (20) et ledit élément tournant (10) permettant dans une position embrayée que ladite première fraise (20) soit liée en rotation avec ledit élément tournant (10) quand ledit élément tournant (10) est mis en rotation et, dans une position de repos que ladite première fraise (20) ne soit pas liée en rotation avec ledit élément tournant (10) quand ledit élément tournant (10) est mis en rotation ;
- des moyens de désengagement (60) de ladite position embrayée vers ladite position de repos; et,
- un carter (40) enveloppant en partie ladite deuxième fraise (30), ladite deuxième fraise (30) étant liée audit carter (40) par une liaison pivot glissant (70) et comprenant une surface d'appui (32) apte à venir en butée contre ledit carter (40) lorsque ladite deuxième fraise (30) est animée d'un mouvement de translation s'éloignant dudit élément tournant selon ledit axe (100),
**caractérisé en ce que**
ladite première fraise (20) comprend au moins une première surface d'appui (22) apte à venir en butée contre ladite deuxième fraise (30) lorsque ladite première fraise (20) est mise en rotation autour dudit axe (100) et, au moins une deuxième surface d'appui (23) apte à venir en butée contre ladite deuxième fraise (30) lorsque ladite première fraise (20) est animée d'un mouvement de translation s'éloignant dudit élément tournant (10) selon ledit axe (100);
et **en ce que**
ledit carter est fixé en l'une de ses extrémités audit élément tournant (10).

2. Perforateur crânien (1) selon la revendication 1, **caractérisé en ce que** ledit carter (40) comprend une section cylindrique d'un diamètre supérieur au diamètre d'une section cylindrique de la deuxième fraise (30), ladite liaison pivot glissant (70) étant formée par au moins une lèvre (71) faisant saillie sur une surface extérieure de ladite section cylindrique de ladite deuxième fraise (30) ou sur une surface intérieure de ladite section cylindrique dudit carter (40).

3. Perforateur crânien (1) selon la revendication 2, **caractérisé en ce que** ladite liaison pivot glissant (70) est formée par deux lèvres (71) faisant saillie sur une surface extérieure de ladite section cylindrique de ladite deuxième fraise (30) ou sur une surface intérieure de ladite section cylindrique dudit carter (40).

4. Perforateur crânien (1) selon l'une quelconque des revendications précédentes, **caractérisé en ce que** ledit carter (40) est fixé audit élément tournant (10) par un emboîtage élastique irréversible dudit carter (40) avec ledit élément tournant (10).

5. Perforateur crânien (1) selon l'une quelconque des revendications précédentes, **caractérisé en ce que** lesdits moyens de désengagement (60) comprennent un moyen de rappel, tel un ressort de compression (61), qui tend à ramener ladite première fraise (20) dans ladite position de repos.

6. Perforateur crânien (1) selon la revendication 5, **caractérisé en ce que** lesdits moyens de désengagement (60) comprennent ladite au moins une première surface d'appui (22) de ladite première fraise (20) et, au moins une rampe (33) disposée sur ladite deuxième fraise (30) et inclinée par rapport audit axe (100),
ladite au moins une première surface d'appui (22) de ladite première fraise (20) apte à venir en butée contre ladite deuxième fraise (30), étant apte à former une liaison glissière lorsque ladite première fraise (20) est mise en rotation.

7. Perforateur crânien (1) selon la revendication 6, **caractérisé en ce que** l'angle formé entre ladite au moins une rampe (33) et ledit axe (100) est compris entre 15° et 75° par rapport audit axe (100), préférentiellement entre 30° et 60° par rapport audit axe (100), de manière davantage préférée égale à 45° par rapport audit axe (100).

8. Perforateur crânien (1) selon l'une quelconque des revendications 6 ou 7, **caractérisé en ce que** ladite première fraise (20) comprend deux premières surfaces d'appui (22) aptes à former deux liaisons glissières avec deux rampes (33) disposées sur ladite deuxième fraise (30), les deux rampes (33) et, respectivement les deux premières surfaces d'appui (22), ne se superposant pas par rotation d'un angle de 180° selon ledit axe (100).

9. Perforateur crânien (1) selon l'une quelconque des revendications précédentes, **caractérisé en ce que** lesdits moyens d'embrayage (50) entre ladite première fraise (20) et ledit élément tournant (10) comprennent au moins un plan incliné (11) et une surface de blocage (12) disposés sur l'un de ladite première fraise (20) ou dudit élément tournant (10) et au moins un doigt (24) présentant une surface d'appui (25) susceptible de venir en contact avec ladite surface de blocage (12) dans ladite position d'embrayage, ledit au moins un doigt (24) étant disposé sur l'autre de ladite première fraise (20) ou dudit élément tournant (10).

10. Perforateur crânien (1) selon l'une quelconque des revendications précédentes, ledit perforateur crânien (1) ne comprenant pas de zone filetée.

## Patentansprüche

1. Schädelbohrer (1), umfassend:
- ein sich drehendes Element (10), das dazu geeignet ist, an einem Antriebselement befestigt zu werden;
- einen ersten Bohrer (20) und einen zweiten Bohrer (30), die dazu geeignet sind, sich um dieselbe Achse (100) zu drehen und um sich translatorisch entlang der besagten Achse (100) in Bezug aufeinander sowie jeweils in Bezug auf das besagte sich drehende Element (10) zu bewegen, und die jeweils an einem ihrer Enden eine Vielzahl von Zähnen (21; 31) aufweisen,
wobei der besagte erste Bohrer (20) im Inneren des besagten zweiten Bohrers (30) aufgenommen ist,
- Kupplungsmittel (50), die auf dem besagten ersten Bohrer (20) und dem besagten sich drehenden Element (10) angeordnet sind, und die es in einer eingekuppelten Position gestatten, dass der besagte erste Bohrer (20) rotatorisch mit dem besagten sich drehenden Element (10) verbunden ist, wenn das besagte sich drehende Element (10) in Rotation versetzt wird, und in einer Ruheposition, dass der besagte erste Bohrer (20) nicht rotatorisch mit dem besagten sich drehenden Element (10) verbunden ist, wenn das besagte sich drehende Element (10) in Rotation versetzt wird;
- Mittel (60) zum Lösen aus der besagten eingekuppelten Position in Richtung der besagten Ruheposition; und
- ein Gehäuse (40), das teilweise den besagten zweiten Bohrer (30) umhüllt, wobei der besagte zweite Bohrer (30) mit dem besagten Gehäuse (40) durch eine gleitende Schwenkverbindung (70) verbunden ist und eine Anlagefläche (32) umfasst, die dazu geeignet ist, in Anlage gegen das besagte Gehäuse (40) zu gelangen, wenn der besagte zweite Bohrer (30) von einer Translationsbewegung geführt wird, indem er sich von dem besagten sich drehenden Element in der besagten Achse (100) entfernt,
**dadurch gekennzeichnet, dass**
der besagte erste Bohrer (20) mindestens eine erste Anlagefläche (22) umfasst, die dazu geeignet ist, gegen den besagten zweiten Bohrer (30) in Anlage zu gelangen, wenn der besagte erste Bohrer (20) um die besagte Achse (100) in Rotation versetzt wird, und mindestens eine zweite Anlagefläche (23), die dazu geeignet ist, gegen den besagten zweiten Bohrer (30) in Anlage zu gelangen, wenn der besagte erste Bohrer (20) von einer Translationsbewegung geführt wird, indem er sich von dem besagten sich drehenden Element (10) in der besagten Achse (100) entfernt,
und dadurch, dass
das besagte Gehäuse an einem seiner Enden an dem besagten sich drehenden Element (10) befestigt ist.

2. Schädelbohrer (1) nach Anspruch 1, **dadurch gekennzeichnet, dass** das besagte Gehäuse (40) einen zylindrischen Querschnitt mit einem Durchmesser umfasst, der größer ist als der Durchmesser eines zylindrischen Querschnitts des zweiten Bohrers (30), wobei die besagte gleitende Schwenkverbindung (70) aus mindestens einem Rand (71) gebildet ist, der von einer Außenfläche des besagten zylindrischen Querschnitts des besagten zweiten Bohrers (30) oder von einer Innenfläche des besagten zylindrischen Querschnitts des besagten Gehäuses (40) vorsteht.

3. Schädelbohrer (1) nach Anspruch 2, **dadurch gekennzeichnet, dass** die besagte gleitende Schwenkverbindung (70) aus zwei Rändern (71) gebildet ist, die von einer Außenfläche des besagten zylindrischen Querschnitts des besagten zweiten Bohrers (30) oder von einer Innenfläche des besagten zylindrischen Querschnitts des besagten Gehäuses (40) vorstehen.

4. Schädelbohrer (1) nach einem der vorhergehenden Ansprüche, **dadurch gekennzeichnet, dass** das besagte Gehäuse (40) an dem besagten sich drehenden Element (10) durch eine elastische irreversible Einpassung des besagten Gehäuses (40) an dem besagten sich drehenden Element (10) befestigt ist.

5. Schädelbohrer (1) nach einem der vorhergehenden Ansprüche, **dadurch gekennzeichnet, dass** die besagten Mittel (60) zum Lösen ein Rückholmittel, etwa eine Druckfeder (61), umfassen, das darauf hinwirkt, den besagten ersten Bohrer (20) in die besagte Ruheposition zurückzuführen.

6. Schädelbohrer (1) nach Anspruch 5, **dadurch gekennzeichnet, dass** die besagten Mittel (60) zum Lösen die besagte mindestens eine Anlagefläche (22) des besagten ersten Bohrers (20) und mindestens eine Rampe (33) umfassen, die auf dem besagten zweiten Bohrer (30) angeordnet und relativ zu der besagten Achse (100) geneigt ist,
wobei die besagte mindestens eine Anlagefläche (22) des besagten ersten Bohrers (20) dazu geeignet ist, gegen den besagten zweiten Bohrer (30) in Anlage zu gelangen, wobei sie dazu geeignet ist, eine Gleitschienenverbindung zu bilden, wenn der besagte erste Bohrer (20) in Rotation versetzt wird.

7. Schädelbohrer (1) nach Anspruch 6, **dadurch gekennzeichnet, dass** der zwischen der besagten mindestens einen Rampe (33) und der besagten Achse (100) gebildete Winkel zwischen 15° und 75° relativ zu der besagten Achse (100) beträgt, vorzugsweise zwischen 30° und 60° in Bezug auf die besagte Achse (100), vorteilhaft bevorzugt gleich 45° in Bezug auf die besagte Achse (100).

8. Schädelbohrer (1) nach einem der Ansprüche 6 oder 7, **dadurch gekennzeichnet, dass** der besagte erste Bohrer (20) zwei erste Anlageflächen (22) umfasst, die dazu geeignet sind, zwei Gleitschienenverbindungen mit zwei Rampen (33) zu bilden, die auf dem besagten zweiten Bohrer (30) angeordnet sind, wobei die beiden Rampen (33) und jeweils die beiden ersten Anlageflächen (22) einander nicht durch Drehung unter einem Winkel von 180° in der besagten Achse (100) überlagern.

9. Schädelbohrer (1) nach einem der vorhergehenden Ansprüche, **dadurch gekennzeichnet, dass** die besagten Kupplungsmittel (50) zwischen dem besagten ersten Bohrer (20) und dem besagten sich drehenden Element (10) mindestens eine geneigte Ebene (11) und eine Blockierungsfläche (12), die auf einem von dem besagten ersten Bohrer (20) oder dem besagten sich drehenden Element (10) angeordnet sind, und mindestens einen Finger (24) umfassen, der eine Anlagefläche (25) aufweist, die mit der besagten Blockierungsfläche (12) in der besagten Kupplungsposition in Kontakt gelangen kann, wobei der mindestens eine Finger (24) auf dem anderen von dem besagten zweiten Bohrer (20) oder dem besagten sich drehenden Element (10) angeordnet ist.

10. Schädelbohrer (1) nach einem der vorhergehenden Ansprüche, wobei der besagte Schädelbohrer (1) keinen Gewindebereich umfasst.

## Claims

1. Cranial perforator (1) comprising:
- a rotating element (10) capable of being fixed to a driving element;
- a first drill-head (20) and a second drill-head (30) capable of rotating about a same axis (100) and of moving in translation along said axis (100) relative to each other and each relative to said rotation element (10) and each having, at one of its extremities, a plurality of teeth (21; 31),
said first drill-head (20) being housed within said second drill-head (30),
- clutch means (50) disposed on said first drill-head (20) and said rotating element (10) making it possible, in an engaged position, for said first drill-head (20) to be linked in rotation with said rotating element (10) when said rotating element (10) is put into rotation and making it possible, in an idle position, for said first drill-head (10) not to be linked in rotation with said rotating element (10) when said rotating element (10) is put into rotation;
- means of disengagement (60) from said engaged position to said idle position; and
- a casing (40) partly encasing said second drill-head (30), said second drill-head (30) being linked to said casing (40) by a sliding pivot link (70) and comprising a supporting surface (32) capable of abutting said casing (40) when said second drill-head (30) is driven by a movement of translation moving away from said rotating element along said axis (100);
**characterized in that**:
said first drill-head (20) comprises at least one first supporting surface (22) capable of abutting said second drill-head (30) when said first drill-head (20) is put into rotation about said axis (100) and, at least one second supporting surface (23) capable of abutting said second drill-head (30) when said first drill-head (20) is driven by a motion of translation moving away from said rotating element (10) along said axis (100);
and **in that**
said casing is fixed at one of its extremities to said rotating element (10).

2. Cranial perforator (1) according to claim 1, **characterized in that**
said casing (40) comprises a cylindrical section with a diameter greater than the diameter of a cylindrical section of the second drill-head (30), said sliding pivot link (70) being formed by two lips (71) protruding out of an external surface of said cylindrical section of said second drill-head (30) or an internal surface of said cylindrical section of said casing (40).

3. Cranial perforator (1) according to claim 2, **characterized in that** said sliding pivot link (70) is formed by two lips (71) protruding out of an external surface of said cylindrical section of said second drill-head (30) or out of an internal surface of said said cylindrical section of said casing (40).

4. Cranial perforator (1) according to any one of the preceding claims, **characterized in that** said casing (40) is fixed to said rotating element (10) by an irreversible elastic interlocking of said casing (40) with said rotating element (10).

5. Cranial perforator (1) according to any one of the preceding claims, **characterized in that** said disengagement means (60) comprise a return means, such as a compression spring (61), tending to bring said first drill-head (20) back into said idle position..

6. Cranial perforator (1) according to claim 5, **characterized in that** said means of disengagement (60) comprise said at least one first supporting surface (22) of said first drill-head (20) and at least one ramp (33) disposed on said second drill-head (30) and inclined relative to said axis (100),
said at least one first supporting surface (22) of said first drill-head (20), capable of abutting said second drill-head (30), being capable of forming a sliding link when said first drill-head (20) is put into rotation.

7. Cranial perforator (1) according to claim 6, **characterized in that** the angle formed between said at least one ramp (33) and said axis (100) ranges from 15° to 75° relative to said axis (100) preferably from 30° to 60° relative to said axis (100), and is more preferably equal to 45° relative to said axis (100).

8. Cranial perforator (1) according to any one of the claims 6 or 7, **characterized in that** said first drill-head (20) comprises two first supporting surfaces (22) capable of forming two sliding links with two ramps (33) disposed on said second drill-head (30) and the two ramps (33) and the two supporting surfaces (22) respectively do not get superimposed by rotation by an angle of 180° along said axis (100).

9. Cranial perforator (1) according to any one of the preceding claims, **characterized in that** said clutch means (50) between said first drill-head (20) and said rotating element (10) comprise at least one inclined plane (11) and a blocking surface (12) disposed on one of said drill-head (20) and rotating element (10), and at least one finger (24) having a supporting surface (25) capable of coming into contact with said blocking surface (12) in said engaged position, said at least one finger (24) being disposed on the other of said first drill-head (20) or said rotating element (10).

10. Cranial perforator (1) according to any one of the preceding claims, said cranial perforator (1) comprising no threaded zone.
